Europäisches Patentamt

European Patent Office

Office européen des brevets

(11) Publication number: **0 404 729**
**A1**

## EUROPEAN PATENT APPLICATION

(21) Application number: **90810452.4**

(51) Int. Cl.⁵: **C12N 15/01, A01K 67/02**

(22) Date of filing: **20.06.90**

(30) Priority: **20.06.89 JP 157469/89**

(43) Date of publication of application:
**27.12.90 Bulletin 90/52**

(84) Designated Contracting States:
**DE FR GB IT**

(71) Applicant: **NIPPON SHINYAKU CO., LTD.**
**14, Kisshoinnishinoshomonguchicho**
**Minami-ku Kyoto-shi Kyoto 601(JP)**

(72) Inventor: **Miyawaki, Shigeki**
**3-D-311, 151-30 Ninomaru-cho, Mukaijima**
**Fushimi-ku, Kyoto 612(JP)**
Inventor: **Yoshida, Hirotsugu**
**33-1 Yoshida Honmachi, Sakyo-ku**
**Kyoto 606(JP)**
Inventor: **Nishi, Toyoyuki**
**35 Nishimachi**
**Kameoka, Kyoto 621(JP)**

(74) Representative: **Kovacs, Paul et al**
**NOVAPAT - CABINET CHEREAU 9, rue du**
**Valais**
**CH-1202 Genève(CH)**

(54) **A new strain of mice.**

(57) A disease susceptible mouse which is characterized by the presence of a pair of alymphoplasia mutant genes resulting in a mouse useful as a model in research on development, differentiation and immune function.

EP 0 404 729 A1

# A NEW STRAIN OF MICE

The present invention relates to a new strain of mice. The mice according to the present invention lack general lymph nodes and Peyer's patches and are accompanied by immunodeficiency and ataxia. Consequently, they are extremely useful for medical research and investigations on development, differentiation and immune function of the lymphatic tissue.

In order to study a particular disease, it is necessary to use an experimental animal which manifests the disease in question. It has, therefore, been routine for researchers to maintain animal lines manifesting particular diseases in order to proceed with said experimental investigations. Mice obtained by mutation have been used as models for human diseases for the purpose of investigation of immunodeficiencies. Examples are nu mice, me mice, scid mice, Dh mice, bg mice, xid mice, hr mice, wst mice, lh mice, etc. Among these, athymic nu mice and asplenic Dh mice are highly valued as models for human diseases.

Ataxia telangiectasia, (hereinafter "AT"), is a disorder which includes development of symptoms such as hypoplasia of thymus, progressive ataxia, immunodeficiency, etc. One disadvantage is that up until this point, there has been no model for collectively studying these symptoms. Another disadvantage has been that immunodeficiency involves various modes and the mechanism of development is complicated. It therefore cannot be asserted that any relationship between cause and development of immunodeficiency has been positively recognized. It is therefore one object of the present invention to seek an ideal mode of disease model for immunodeficiency and compensate for the above described defect by obtaining a more complete model animal for human diseases. The present invention provides such a model.

The mice of the present invention lack general lymph nodes and are highly susceptible to infection. Accordingly, such mice provide a powerful tool for the study of development and differentiation of peripheral lymphatic tissue and the relationship between peripheral lymphatic tissue and local immune response.

Mice of the present invention are homozygous for a novel mutant recessive gene, hereinafter called alymphoplasia, (aly). A mouse homozygous for the recessive aly gene is hereinafter referred to as a "mutant" mouse. Mice which are termed "heterozygotes" have a genotype of aly/ + , and are phenotypically "normal" in appearance. Mice with this genotype are hereinafter be referred to as "heterozygotes". These mice are carriers for the aly gene, but are themselves healthy in appearance. Mice containing a pair of dominant + genes, are referred to as normal homozygous mice.

Mice of the present invention are characterized by the presence of a pair of the recessive alymphoplasia genes.

Mice of the present invention are further characterized by the absence of general lymph nodes.

Mice of the present invention are further characterized by a susceptibility to infection or immunodeficiency.

Mice of the present invention are further characterized by the presence of the disorder resembling ataxia telangiectasia.

A mutant mouse line of the present invention may be obtained by the following procedure. Inbred C57BL/6J strain of mice (obtained from the Jackson Laboratory, USA, in 1973) had been maintained in the Yamashina Laboratories of Nippon Shinyaku Co., Ltd. In 1986, the present inventors found several mice which exhibited slowed motion from about 3 months of age, and which eventually developed hind limb paralysis.

Since the C57BL/6J strain has poor reproductive performance, it was difficult to maintain this abnormal characteristic during the course of breeding. Thus, in order to improve the reproductive performance using hybrid vigor (heterosis), one male mouse assumed to be a heterozygote was mated with a female AEJ/GnRk mouse. The AEJ/GnRk strain was obtained from the Jackson Laboratory, USA, in 1987. This strain has extremely high reproductive performance. The young obtained from this mating were assumed to be heterozygous for the mutant gene and homozygous for the normal gene in a ratio of approximately 1:1. Mating was random between the young mice and offspring considered to be a result of mating between heterozygotes were selected.

It is believed that matings which produced mice with the abnormality of the present invention (i.e., mice considered to be homozygotes for the mutant gene) would be those that occurred between the heterozygotes. The statistical probability of the result of the matings of such combination is theoretically 1/2 x 1/2 = 1/4. In practice, among 19 pairs of matings made, 3 pairs produced abnormal progeny. They are considered to be matings of the heterozygotes inter se.

From the mating between the heterozygotes, homozygotes for the mutant gene, heterozygotes and homozygotes for the normal gene would be obtained in a ratio of 1 : 2 : 1. Accordingly, it was attempted to

2

fix the mutant characteristic by mating mutant homozygotes, using young mice obtained from matings between those believed to be heterozygotes. Since it was assumed that the animal would not breed after development of ataxia, it was attempted to randomly mate the mice prior to development of ataxia and select the combination of mating between the mutants (which were identified by developing the symptom later). This procedure reveals that male mutant mice are capable of breeding until ataxia is developed, but mutant female mice are rarely fertile and even though a very few mice get pregnant, they lack nursing ability.

After examining the line containing the mutation, it was concluded that said mutation is controlled by a single autosomal recessive gene. As will be described hereinafter, this can be proven by a mating experiment. The progeny of matings between female heterozygotes (aly/+) and male mutants (aly/aly) were selected in order to perform pedigree breedings. Among the combinations of mating between apparently normal female mice and male mice with ataxia, ie. aly/aly, the combination in which mice with the aly/aly mutation appeared is the desired mating combination. Where the young mice obtained from the mating between the heterozygotes were mated at random, a probability of mating between female heterozygotes and males homozygous for the aly gene is theoretically 1/2 X 1/4 = 1/8. In actual practice, one pair of the desired mating combination could be obtained for every mating of 13 pairs.

From the mating between the female heterozygote, aly/+, and the male homozygous for the aly gene, the aly/+ mice and the aly/aly mice were obtained in a ratio of 1 : 1. When they are mated at random, a possibility of mating between aly/+ female mice and aly/aly male mice is 1/2 X 1/2 = 1/4. By repeating the same procedure, the aly gene is preserved with certainty, thus obtaining mice of the present invention (ie. homozygous aly mutant mice). The present invention has also succeeded in acquiring a strain of mice with improved reproduction ability (heterosis), by crossing aly mice with the AEJ/GnRk strain of mice. This makes it possible to maintain and produce a stable population of mice of the present invention.

Maintenance of the strain of mice of the present invention can be accomplished as follows. Mice of the present invention are homozygotes for the aly gene. As has been described above, the female aly/aly mice cannot breed, but the male aly/aly mice are capable of breeding until they develop ataxia. Therefore, in order to maintain a line of mice of the present invention, a female heterozygote, aly/+, is mated with a male homozygote, aly/aly, prior to development of ataxia in each generation. Commonly, mice of the present invention are about 3 to 4 months old when they begin to develop ataxia, but until that time, progeny can be obtained 1 to 3 times. About 9 mice can be obtained by one delivery, with most of them growing until they are weaned.

Aly/+ mice and aly/aly mice of the present invention are obtained in a ratio of 1 : 1 from the mating between female aly/+ mice and male aly/aly mice. A random mating between them gives a mating combination of female aly/+ mice and male aly/aly mice again with a probability of 1/4. Using the young mice obtained from this mating combination, further mating is performed to obtain the next generation with the above procedure being repeated. Mating approximately 12 pairs in every generation will allow the strain to be well maintained.

Another method of producing the mice of the present invention is as follows. The mice of the present invention can be efficiently produced by utilizing ovarian transplantation techniques. While female aly/aly homozygotes (i.e. mice of the present invention) cannot breed, their ovaries function normally when transplanted to a normal (+/+) mouse.

Accordingly, when the ovary from the female aly/aly mouse is transplanted to a histocompatible and normal (ie. +/+) mouse and the male aly/+ mouse is mated with this transplanted female mouse, fertility results are similar to those obtained when producing normal (+/+) mice, as the male aly/+ mouse is capable of breeding up to about 18 months old, as are normal (+/+) mice, unlike the male aly/aly mouse.

Aly/aly mice and aly/+ mice are obtained in a ratio of 1 : 1 by this mating and apparently phenotypically cannot be distinguished prior to development of ataxia (anatomically, confirmation can be made by the presence or absence of lymph nodes).

To obtain aly/aly mice alone, a normal (+/+) female mouse with ovaries transplanted from a female aly/aly mouse is mated with a male aly/aly mouse. In this case, however, the male aly/aly mouse can breed only until it reaches 3 to 4 months of age, thus productivity is poor.

Characteristics of the Mice of the Present Invention

I. Genetic Analysis

In order to breed mice of the present invention, mating experiments were performed using young believed to be homozygous aly/aly and heterozygous aly/+ in a ratio of 1 : 1. These mice were obtained from matings considered to be between mutant mice (ie., aly homozygotes) and carrier mice (heterozygotes i.e. phenotypically normal). The results of these mating experiments are shown in Table 1. These experiments establish that the mutation of the present invention is controlled by a single autosomal recessive gene. The most outstanding and unique characteristic of mice of the present invention is their lack of general lymph nodes.

Table 1

| Mating | | Offspring | | | | | |
| --- | --- | --- | --- | --- | --- | --- | --- |
| | | Normal | | | Abnormal | | |
| Female | Male | Female | Male | Total | Female | Male | Total |
| I normal (aly/ + ) | abnormal (aly/aly) | 33 | 24 | 57 | 25 | 26 | 51 |
| II normal (aly/ + ) | normal (aly/ + ) | 64 | 70 | 134 | 19 | 25 | 44 |

II. Characteristics of the Diseases

Diseases in the mice of the present invention are characterized by the following properties.

(1) Aplasia of lymphatic tissue

(i) The presence of general lymph nodes such as mesenteric lymph nodes, submaxillary lymph nodes, inguinal lymph nodes, etc. and Payer's patch are not observed under a dissecting microscope.

(ii) The thymus shows hypoplasia more or less (reaching about only 50% of the weight as compared to a normal control) from the time of weaning (3 weeks old). As the disease progresses, the thymus undergoes marked atrophy and occasionally is not perceptible with the naked eye. Histologically, there seems to be a defect in the thymic medulla.

(iii) Spleen weight is almost the same as in the control and no difference in cell count has been noted. However, histologically, clear follicle formation is not recognized. Analysis of lymphocyte surface antigens reveals that the ratio of B cells is significantly lower than normal control (50 to 60% as compared to the normal control).

(2) Ataxia

The mice of the present invention develop progressive ataxia. In many cases, ataxia can appear either before or after about 3 months of age. The symptoms consist of slow motion or a lack of quick action, which then progresses to gait instability and finally results in paralysis of hind limbs. This occurs from 4 to 5 months of age. Mice with paralysis of the hind limbs generally die within 2 weeks.

(3) Other Diseases

In mice of the present invention, symptoms suggesting high susceptibility to infection (immunodeficiency) such as intestinal mucosa hypertrophy, focal necrosis of hepatic cells, subcutaneous abscess, etc. are observed. Furthermore, a high frequency of reticuloendothelial neoplasia is noted (about 10%).

The following nonlimitative example describes the breeding of the mice according to the present invention.

EXAMPLE

Ovaries from female mice 3 to 4 months old were transplanted to 10 (C57BL/6J X AEJ/GnRk)F₁ mice 45 to 60 days old. The ovary transplant was performed by completely replacing the ovaries in both ovarian capsules in the recipient mice with the ovaries of the donor mice. After a recovery period of from 10 to 15 days the female mice were mated with heterozygote male mice approximately 4 months old. The mating was performed by housing together one pair of female and male mice in a plastic cage (20 cm (W) x 30 cm (d) x 13 cm (H)) throughout the breeding period (approximately 1 year).

Seven mice out of the 10 ovary-transplanted mice underwent breeding and littered down 5.7 times on the average (40 times in total) to give 237 mice. Out of the 237 mice, 226 grew and 109 out of the 226 showed abnormal characteristics. Therefore, on the average, about 11 mutant mice could be produced for every ovary transplantation.

Since mice of the present invention are bred by cross breeding the C57BL/6J strain and the AEJ/GnRk strain, the (C57BL/6J x AEJ/GnRk)F₁ mice are histocompatible, and hence, the (C57BL/6J x AEJ/GnRk)F₁ female mice were used as ovary transplant recipients.

The results of breeding the ovary-transplanted animal are shown in Table 2.

Table 2

| Total Mouse No. | Number of Delivery | Number of Mice Born | Number of Weaned Mice | | |
|---|---|---|---|---|---|
| | | | Normal | Abnormal | Total |
| 1 | 6 | 40 | 19 | 17 | 36 |
| 2 | 0 | -- | -- | -- | -- |
| 3 | 8 | 44 | 23 | 21 | 44 |
| 4 | 4 | 21 | 9 | 11 | 20 |
| 5 | 0 | -- | -- | -- | -- |
| 6 | 4 | 27 | 13 | 13 | 26 |
| 7 | 5 | 28 | 13 | 12 | 25 |
| 8 | 6 | 30 | 14 | 16 | 30 |
| 9 | 0 | -- | -- | -- | -- |
| 10 | 7 | 47 | 26 | 19 | 45 |
| Total | 40 | 237 | 117 | 109 | 226 |

## Claims

1. A disease susceptible mouse characterized by the presence of a pair of alymphoplasia mutant genes.

2. The mouse according to claim 1, further characterized by an absence of general lymph nodes.

3. The mouse according to claim 1, further characterized by susceptibility to infection.

4. The mouse according to claim 1, further characterized by ataxia.

5. The mouse according to claim 1, further characterized by atrophy of the thymus.

6. The mouse according to claim 1, further characterized by immunodeficiency.

7. The mouse according to claim 6, further characterized by intestinal mucosa hypertrophy.

8. The mouse according to claim 6, further characterized by focal necrosis of hepatic cells.

9. The mouse according to claim 6, further characterized by subcutaneous abscesses.

10. The mouse according to claim 6, further characterized by a high frequency of reticuloendothelial neoplasia.

11. A disease susceptible mouse characterized by the presence of a pair of alymphoplasia mutant genes produced by breeding a C57BL/6J mouse with a AEJ/GnRk mouse, wherein said C57BL/6J mouse is heterozygous for said mutant gene;

wherein an F₁ generation of heterozygotes for said mutant gene were allowed to randomly mate obtaining mice homozygous for said mutant gene; and

wherein said mutant homozygous mice were mated with heterozygotes prior to development of ataxia in said homozygotes.

12. A disease susceptible mouse characterized by the presence of a pair of alymphoplasia mutant genes produced by crossing a female heterozygote mouse with a male homozygote mutant mouse, prior to development of ataxia in said male.

13. A disease susceptible mouse characterized by the presence of a pair of alymphoplasia mutant genes produced by transplanting ovaries from a female homozygote mutant mouse to a histocompatible mouse lacking alymphoplasia genes and mating said histocompatible female mouse with a male mouse homozygous for said mutant gene wherein said male mouse is mated prior to development of ataxia.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | NATURE. vol. 297, 03 June 1982, LONDON GB pages 402 - 404; SHULTZ, L.D. et al.: ""Wasted", a new mutant of the mouse with abnormalities characteristic of ataxia telangiectasia" * the whole document * | 1-6 | C12N15/01 A01K67/02 |
| X | Protides Biol. Fluids vol. 32, 1985, pages 103 - 106; KAISERLIAN,D & BACH, J.F.: "THE WASTED MUTANT MOUSE : a model for the study of IgA deficiency in ataxia-telangiectasia" * figure 1 * | 1-6 | |

TECHNICAL FIELDS SEARCHED (Int. Cl.5 )

C12N
A01K

The present search report has been drawn up for all claims

| Place of search | Date of completion of the search | Examiner |
|---|---|---|
| THE HAGUE | 03 OCTOBER 1990 | CHAMBONNET F.J. |